# EUROPEAN PATENT SPECIFICATION

(11) **EP 2 577 312 B1**
(45) Date of publication and mention of the grant of the patent: **30.08.2017**
(21) Application number: 11790355.9
(22) Date of filing: 01.06.2011
(51) Int. Cl.: A61K 31/22, G01N 33/68, G01N 33/566, G01N 33/92, A61K 31/277, A61K 31/366, A61K 31/40, A61K 31/4365, A61K 31/554

(54) **PREDICTING PLAQUE ACCUMULATION**
VORHERSAGE EINER PLAQUE-AKKUMULIERUNG
PRÉDICTION DE L'ACCUMULATION DE LA PLAQUE

(30) Priority: 01.06.2010 US 350304 P
(43) Date of publication of application: 10.04.2013
(73) Proprietor: Boston Heart Lab, Framingham, MA 01701 (US)
(72) Inventor: SCHAEFER, Ernst, J., Framingham, MA 01760 (US); ASZTALOS, Bela, F., Framingham, MA 01760 (US)
(74) Representative: Kirkham, Nicholas Andrew
(86) International application number: PCT/US2011/038801
(87) International publication number: WO 2011/153271

(56) References cited:
- US-A1- 2007 196 841
- US-A1- 2010 076 787
- B. F. ASZTALOS: "Value of High-Density Lipoprotein (HDL) Subpopulations in Predicting Recurrent Cardiovascular Events in the Veterans Affairs HDL Intervention Trial", ARTERIOSCLEROSIS, THROMBOSIS, AND VASCULAR BIOLOGY, vol. 25, no. 10, 1 October 2005 (2005-10-01), pages 2185-2191, XP055075083, Dallas TX USA ISSN: 1079-5642, DOI: 10.1161/01.ATV.0000183727.90611.4f
- ASZTALOS B F ET AL: "LpA-I, LpA-I:A-II HDL and CHD-risk: The Framingham Offspring Study and the Veterans Affairs HDL Intervention Trial", ATHEROSCLEROSIS, vol. 188, no. 1, 1 September 2006 (2006-09-01), pages 59-67, XP028071474, ELSEVIER IRELAND LTD, Dublin ISSN: 0021-9150, DOI: 10.1016/J.ATHEROSCLEROSIS.2005.10.018 [retrieved on 2006-09-01]
- L. CAMONT ET AL: "Biological activities of HDL subpopulations and their relevance to cardiovascular disease", TRENDS IN MOLECULAR MEDICINE, vol. 17, no. 10, 1 October 2011 (2011-10-01), pages 594-603, XP028307018, Cell Press Elsevier Inc,location Cambridge, MA USA ISSN: 1471-4914, DOI: 10.1016/J.MOLMED.2011.05.013 [retrieved on 2011-06-03]
- DAVID C. ISBELL ET AL: "Reproducibility and Reliability of Atherosclerotic Plaque Volume Measurements in Peripheral Arterial Disease with Cardiovascular Magnetic Resonance", JOURNAL OF CARDIOVASCULAR MAGNETIC RESONANCE, vol. 9, no. 1, 1 January 2007 (2007-01-01) , pages 71-76, XP055075125, Informa Healthcare, Zug CH ISSN: 1097-6647, DOI: 10.1080/10976640600843330
- R.P. NOBLE: "Electrophoretic separation of plasma lipoproteins in agarosen gel", J LIPID RES (AMERICAN SOCIETY FORBIOCHEMISTRY AND MOLECULAR BIOLOGY), vol. 9, 1968, pages 693-700, Rockville MD USA
- B. F. Asztalos ET AL: "Effects of atorvastatin on the HDL subpopulation profile of coronary heart disease patients", Journal of Lipid Research, vol. 43 1 October 2002 (2002-10-01), 2002, pages 1701-1707, XP055215459, Rockville MD United States DOI: 10.1194/jlr.M200037-JLR200 Retrieved from the Internet: URL:http://www.jlr.org/cgi/content/abstrac t/43/10/1701 [retrieved on 2015-09-22]
- Tisha Joy ET AL: "Is raising HDL a futile strategy for atheroprotection?", Nature Reviews. Drug Discovery, vol. 7, no. 2, 1 February 2008 (2008-02-01), pages 143-155, XP055292420, GB ISSN: 1474-1776, DOI: 10.1038/nrd2489

## Description

### TECHNICAL FIELD

The present invention relates to a method for determining a subjects risk of plaque accumulation within a blood vessel, according to claim 1 or claim 5.

### BACKGROUND OF THE INVENTION

Coronary heart disease, the leading cause of morbidity and mortality worldwide, is caused by atherosclerotic plaque deposition in the coronary arteries *(i.e.* atherosclerosis). CHD is a multifactorial disease, and independent risk factors include: age, gender, hypertension, smoking, diabetes, family history of premature CHD, high Low Density Lipoprotein cholesterol (LDL-C) (> 160 mg/dl), and low HDL-C (<40 mg/dl for males and <50 mg/dl for females) levels. However, these established CHD-risk factors account for only about half of the variability in CHD events in the US population. Accumulating data indicate that emerging risk factors, including lipoprotein sub-fractions, are better markers of the disease than many of the established risk factors. Additionally, other factors influence how much the major risk factors affect absolute risk. Randomized intervention trials with statins targeted at lowering LDL-C and CHD risk have established that reductions in LDL-C are associated with about a 30% reduction in cardiovascular disease (CVD) events compared to placebo. This evidence suggests that other modalities, to lower CVD morbidity and mortality in patients with established CHD, need to be evaluated.
While aggressive statin monotherapy has been shown to decrease the rate of CHD progression, significant regression has only been noted with agents that decrease LDL and increase HDL.

A simple paradigm of atherosclerosis is that there is an antagonistic relationship between apolipoprotein-B (apo-B)-containing particles, such as LDL particles, and apoA-I-containing particles, such as HDL particles. For example, apo-B-containing particles promote atherosclerosis *(i.e.* they are atherogenic) because they are deposited on the arterial wall; however, apoA-I-containing particles counteract this effect *(i.e.* they are atheroprotective) because they remove excess cholesterol from the arterial wall.

When removal of excess cholesterol is not able to keep up with the deposition of cholesterol *(i.e.* depositing particles), macrophages turn into foam cells, which in turn set up a maladaptive inflammatory cascade, resulting in the progressive deposition of atherosclerotic material. Early stages of atherosclerosis lead to the deposition of cholesterol, which, with the apoptosis of macrophages, turns into a lipid-rich necrotic core. In the later stages, especially after repeated sub-clinical intra-plaque hemorrhage, there is progressive deposition of calcium through an active process that converts non-calcified plaques into calcified plaques (CAP).

Soft plaque or low-density non-calcified plaque (LD-NCP) is the first grossly visible lesion in the development of atherosclerosis. It appears as an irregular off white to yellow-white discoloration near the luminal surface of the artery. LD-NCP mainly consists of foamy appearing macrophage cells. In the later stages of the disease some, but not all, LD-NCP progresses into fibrous cap forms, which consist of high density non-calcified plaque (HD-NCP).

The deposition of atherogenic lipoprotein particles and the ensuing inflammatory process lead to very specific morphological and compositional changes that can be visualized by a number of different imaging modalities. Early phases of the atherosclerotic process result in deposition of atherosclerotic material in the sub-intima with outward expansion of the external elastic lamina and the preservation of luminal size. Later phases of the disease are characterized by the encroachment of the atherosclerotic plaque on the lumen, leading to luminal stenosis. Geometrical parameters of plaque include measurements of luminal size (minimal lumen diameter (MLD), percent diameter stenosis (%DS), minimal luminal area (MLA), percent area stenosis (%AS)) and plaque size (plaque volume and percent atheroma volume (PAV)). Compositional parameters include the volume and percent of calcified and non-calcified plaque components.

Generally, intravascular ultrasound (IVUS) and contrast-enhanced coronary multi-detector computed tomography angiography (MD-CTA) have been utilized for precise measurement of the geometrical parameters of plaque.

Lipoproteins are complex particles containing various lipid and protein components. Among lipoproteins, HDL is the most complex particle with respect to both composition and function. HDL has several documented functions although the precise mechanism(s) by which it prevents atherosclerosis remains uncertain. HDL can protect against atherosclerosis in several ways. The most cited athero-protective function of HDL is related to its participation in reverse cholesterol transport. During this process, HDL removes cholesterol from macrophages in the vessel wall, preventing the transformation of macrophages into foam cells, thereby preventing the build-up of fatty streaks in the vessel wall. The cholesterol that originated in the macrophages is then carried by HDL into the liver for ultimate excretion into the bile. HDL is also an anti-oxidant and anti-inflammatory agent. Oxidative stress can cause inflammation in the vessel wall which results in increased macrophage recruitment. Macrophages take up oxidized LDL (oxLDL) which ultimately transforms them into foam cells, the first step in plaque formation. HDL's protein and lipid components can prevent LDL oxidation. This is a very important function because oxidized LDL is the major carrier of cholesterol to macrophages present in the vessel wall.

The various HDL subpopulations differ in size and composition, which impart each of the varying HDL subpopulations with different functions and pathophysiological relevance. The many different functions of HDL are not distributed evenly among the various HDL subpopulations. The best illustration of this is the fact that cells have several different ways by which to remove excess cholesterol. Different HDL particles interact with the different pathways specifically depending on the cell type, the expressed receptor protein type on the surface of the cell, and the cellular cholesterol content. Moreover, the different HDL subpopulations participate differently in the anti-oxidation, anti-inflammation, and cell-signaling processes based on the particles' lipid and protein composition.

Most importantly, the HDL subpopulation profile can differentiate subjects with increased risk for CVD from subjects without such risk independently of HDL-C level. This is very important, as some subjects (or even an entire ethnic group) may have low HDL-C levels but present no history of elevated CVD risk. This is due to the fact that these subjects may have not only hyperactive HDL catabolism, but also hyperactive HDL metabolism. However, some subjects with high HDL-C may experience a CVD event due to low HDL metabolism/catabolism or dysfunctional HDL.

Traditionally, intravascular ultrasound (IVUS) and contrast-enhanced coronary multi-detector computed tomography angiography (MD-CTA) has been utilized for precise measurement of plaque geometrical parameters. Disadvantageously, these procedures are invasive, expensive, and subject the patient to significant levels of radiation exposure. Accordingly, there is an urgent need for diagnostic methods that can proactively identify patients with an increased risk of CHD while they are still in the early stages of CHD (i.e. patients with increasing levels of non-calcified plaque).

US 2007/0196841A1 relates to a physiogenomic method for predicting response to diet.

US 2010/0076787A1 relates to a method for preparing a medical data report.
B.F. Asztalos: "Value of High-Density Lipoprotein (HDL) Subpopulations in Predicting Recurrent Cardiovascular Events in the Veterans Affairs HDL Intervention Trial." Arteriosclerosis, Thrombosis, and Vascular Biology. Vol. 24, no.10, 1 October 2005, pages 2185-2191.
B.F. Asztalos, et al: "LpA-I, LpA-I:A-II HDL and CHD-risk: The Framingham Offspring Study and the Veterans Affairs HDL Intervention Trial." Atherosclerosis. Vol. 188, no. 1, 1 September 2006, pages 59-67.
L. Camont et al: "Biological activities of HDL subpopulations and their relevance to cardiovascular disease", Trends in Molecular Medicine. Vol. 17, no. 10, pages 594-603.
D.C. Isbell, et al: "Reproducibility and Reliability of Atherosclerotic Plaque Volume Measurements in Peripheral Arterial Disease with Cardiovascular Magnetic Resonance." Journal of Cardiovascular Magnetic Resonance, no. 1, pages 71-76.
R.P. Noble: "Electrophoretic separation of plasma lipoproteins in agarose gel." Journal of Lipid Research. Vol 9, 1968, pages 693-700.
B.F. Asztalos, et al: "Effects of atorvastatin on the HDL subpopulation profile of coronary heart disease patients." Journal of Lipid Research. Vol. 43, October 2002, pages 1701-1707.

### SUMMARY OF THE INVENTION

The present invention relates to methods for identifying subjects with increased risk for coronary heart disease (CHD).

In one aspect, the invention provides a method of identifying a subject with increased risk of cardiovascular disease (CVD), the method involving providing an HDL subpopulation by separating the ApoA-I-containing HDL particles from a plasma sample from the subject, where the HDL particles include an alpha1-4 (α1-4) subpopulation, a pre-alpha1-4 (pre-α1-4) subpopulation, and a pre-beta1-2 (pre-β1-2) subpopulation, quantitating members of the HDL subpopulation to provide an HDL subpopulation profile, and comparing the HDL subpopulation profile with a reference HDL subpopulation profile value to identify an increased risk, a borderline risk, or a low risk for CVD in the subject.

In one embodiment, the α subpopulation comprises α-1, α-2, α-3, and α-4. There is also described a pre- α subpopulation comprising pre-α-1, pre-α-2, and pre-α-3. In another embodiment, the pre-β subpopulation comprises pre- β-1 and pre- β-2.

In another embodiment, the reference HDL subpopulation profile values include values indicative of an increased risk profile value, a borderline risk profile value, and a low risk profile value.

In one embodiment, the increased risk profile value comprises an α-1 value of <14 and <20 mg/dl, an α-2 value of <27 and <34 mg/dl, an α-3 value of >30 and >30, and an α-4 value of>25 and >25 mg/dl, and a preβ-1 value of >25 and >25 mg/dl in men and women, respectively.

In another embodiment, the borderline risk profile value comprises an α-1 value between 14-20 mg/dl and 20-30 mg/dl, an α-2 value between 27-34 mg/dl and 34-45 mg/dl, an α-3 value between 13.5-30 mg/dl and 13.5-30 mg/dl, and an α-4 value between 17-25 mg/dl and 17-25 mg/dl in men and women, respectively.

In another embodiment, the low risk profile value comprises an α-1 value of >20 and >30 mg/dl, an α-2 value of >34 and >45 mg/dl, an α-3 value of <13.5 and < 13.5 mg/dl, and an α-4 value of <25 and < 25 mg/dl in men and women, respectively.

The HDL subpopulation profile may be further correlated with at least one additional CVD risk factor selected from the group consisting of age, gender, hypertension, smoking, diabetes, family history of CVD, high LDL-C, and apoB, CRP, and LpPLA2 and low HDL-C and apoA-I.

There is described a method of identifying a subject with increased risk of cardiovascular disease (CVD), the method involving providing an HDL subpopulation by separating the ApoA-I-containing HDL particles from a plasma sample from the subject, where the HDL particles comprise four alpha (α) subpopulations, four pre-alpha (pre- α) subpopulations, and two pre-beta (pre-β) subpopulations, quantitating members of the HDL subpopulation to establish an HDL subpopulation ratio between at least two different HDL particles, and comparing the HDL subpopulation ratio with a reference HDL subpopulation ratio, to identify an increased risk, a borderline risk, or a low risk for CVD in a subject.

In one embodiment, the α subpopulation comprises α-1, α-2, α-3, α-4, and preβ-I.

In another embodiment, the reference HDL subpopulation profile comprises at least one ratio value selected from the group consisting of α-1/β-1, α-1/α-3, α-1/α-4, α-1/(α-3 + α-4), α-2/β-1, α-2/α-3, α-2/α-4, and α-2/(α-3 + α-4).

In yet another embodiment, the HDL subpopulation ratio is α-1/β-1, α-1/α-3, α-1/α-4, or α-1/(α-3 + α-4).

In another embodiment, the HDL subpopulation profile is further correlated with at least one additional CYD risk factor selected from the group consisting of age, gender, hypertension, smoking, diabetes, family history of CVD, high LDL-C, apoB, CRP, and LpPLA2 and low HDL-C and apoA-I. Although not in accordance with the invention as claimed also described is a method of monitoring a subject with increased risk of cardiovascular disease (CVD), the method involving providing an HDL subpopulation by separating the ApoA-I-containing HDL particles from a plasma sample from the subject, where the HDL particles include an alpha (α) subpopulation, a pre-alpha (pre-α) subpopulation, and a pre-beta (pre-β) subpopulation, quantitating members of the HDL subpopulation to establish an a subpopulation ratio, and comparing the a subpopulation ratio with a reference a subpopulation ratio, where an altered a subpopulation ratio indicates an altered risk of CYD in the subject. Although not in accordance with the invention as claimed, also described is a method of identifying a subject with increased risk for stenosis or for having high volume of rupture-prone non-calcified plaque, the method involving providing an HDL subpopulation profile by separating the ApoA-T-containing HDL particles from a plasma sample from the subject, where the HDL particles comprise an alpha (α) subpopulation, a pre-alpha (pre-α) subpopulation, and a pre-beta (pre-β) subpopulations, analyzing the HDL subpopulation profile to determine whether the subject has a suboptimal HDL subpopulation profile, wherein the suboptimal profile is characterized by decreased levels of α-1 and α-2, and increased levels of α-3, α-4, and preβ-1, and where the HDL subpopulation profile indicates an increased risk of having stenosis and or non-calcified plaque formation. Although not in accordance with the invention as claimed, further described is a method of treating a subject having cardiovascular disease (CVD), the method involving providing an HDL subpopulation profile by separating the ApoA-I-containing HDL particles from a plasma sample from the subject, where the HDL particles comprise an alpha (α) subpopulation, a pre-alpha (pre-α) subpopulation, and a pre-beta (pre-β) subpopulation, analyzing the LDL population of the subject to determine relative amounts of a small dense LDL fraction and a large less dense LDL fraction, and treating the subject with a therapeutic compound if the ApoA-I content is less than or equal to 20 mg/dL and the small dense LDL fraction is greater than or equal to 20 mg/dL.

The therapeutic compound is Ramipril, Verapamil, Diltiazem, alpha blockers, beta blockers, lsosorbide mononitrate, lsosorbide dinitrate, Furosemide, Atorvastatin, ACE inhibitors, Atorvastatin, Clopidogrel, Pravastatin, Lisinopril, Simvastatin, Prasugrel, and/or Prasugrel. Although not in accordance with the invention as claimed, in another aspect, the invention provides a method of quantitating ApoA-1-containing HDL particles from a plasma sample from a subject to provide a quantitated value for at least one particle from the group that includes an alpha 1-4 subpopulation, a pre-alpha 1-4 subpopulation, and a pre-beta 1-2 subpopulation to thereby provide an HDL subpopulation profile, and comparing the quantitated value to a reference profile to identify an increased, borderline, or low risk of having soft plaques or developing CVD in the sample or subject.

In one embodiment, HDL particle is α-1 and the reference value for increased risk is <14 mg/dl in men and <20 mg/dl in women.

In another embodiment, the HDL particle is α-I and the reference value for borderline risk is 14-20 mg/dl in men and 20-30 mg/dl in women.

In yet another embodiment, the HDL particle is α-1 and the reference value for low risk is >20 mg/dl in men and >30 mg/dl in women. Although not in accordance with the invention as claimed, diagnostic screening tools for assessing carotid artery stenosis, as well as plaque volume and composition, such as Intravascular Ultrasound Angiography (IVUS) and Multi-detector Computed Tomography Angioplasty (MD-CTA), exist. However, IVUS is an invasive procedure and MD-CTA utilizes ionizing radiation and potentially nephrotoxic contrast agents and both procedures are expensive. Moreover, sophisticated imaging procedures are typically confined to tertiary referral centers and cannot be widely applied in the community. Therefore, there is a need for simpler and more cost-effective means for screening patients with high-risk for CHD.

There is described a method for estimating cardiovascular risk posed by high plaque volumes, especially high volumes of LD- NCP in arteries which can lead to rupture and/or thrombi formation. It is worth noting that the majority of myocardial infarction (MI) is caused by a ruptured plaque and/or thrombi formation in the coronary arteries.

There is also described a method for evaluating coronary artery plaques composition/stability via the subfractionation and analysis of HDL-subclass profile. In part, the present invention is based on the realization that certain HDL particle values in a subject are correlated with increased, borderline, or low risk of the presence of soft plaques. Although not in accordance with the invention as claimed, in another aspect there is a method for evaluating the burden of CHD in an individual utilizing the HDL subpopulation map (HDL-map), along with HDL-C, apoA-I, apolipoprotein-B (apo-B), LDL-C, and at least two inflammatory markers like C-reactive protein (CRP) and lipoprotein associated phospholipase A2 (LpPLA2). Although not in accordance with the invention as claimed, there is described methods for evaluating the severity of coronary artery disease in an individual by estimating plaque composition/stability via the subfractionation and analysis of HDL subclasses. Blood testing, MD-CTA, and IVUS were utilized for measuring and quantifying the various parameters necessary for the evaluation.

Other features and advantages of the invention will be apparent from the detailed description, and from the claims.

### Definitions

The term "apolipoprotein A-I (apoA-I)," as used herein, refers to the measure of the most abundant protein component of HDL with a molecular weight of 28 kilo Daltons (KD). ApoA-I is an essential component of HDL.

The term "atherosclerosis" refers to a condition in which an artery wall thickens as the result of a build-up of fatty materials such as cholesterol. It is a syndrome affecting arterial blood vessels, a chronic inflammatory response in the walls of arteries, in large part due to the accumulation of macrophage white blood cells and promoted by low-density lipoproteins (plasma proteins that carry cholesterol and triglycerides) without adequate removal of fats and cholesterol from the macrophages by functional high density lipoproteins (HDL). It is commonly referred to as a hardening or furring of the arteries. It is caused by the formation of multiple plaques within the arteries.

The term "α-1 HDL particle (α-1)," as used herein, refers to the apoA-I concentration in HDL particles with a modal diameter of 11 nm, as determined by non-denaturing polyacrylamide gel electrophoresis and the use of molecular size standards. Alpha-1 HDL is similar to HDL2, HDL2b, and large HDL separated by other techniques. It is one of the most important HDL particles for predicting heart disease. This is a large, lipid-rich HDL particle containing apoA-I, a large number of free cholesterol and phospholipids (PL) on the surface, and cholesterol ester and triglyceride (TG) in the core. This particle interacts with scavenger receptor BI (SRBI) in the liver and delivers cholesterol into the bile. A decreased α-1 level marks an inadequate HDL metabolism and is associated with increased risk for cardiovascular disease (CVD). Low concentration of α-1 (< 14 mg/dl in men and < 20 mg/dl in women) is associated with significantly increased heart disease risk. An α-1 concentration value between 14mg/dl and 20 mg/dl in men, and between 20 mg/dl and 30 mg/dl in women, is considered borderline. An α-1 concentration value above 20 mg/dl in men and above 30 mg/dl in women is considered optimal.

The term "α-2 HDL particle (α-2)," as used herein, refers to an important HDL particle for predicting heart disease. This HDL particle is medium-large and contains both apoA-I and apoA-II molecules, as well as surface and core lipids. Separated by other methods α-2 is referred to as HDL2a or is incorporated into the medium size HDL subfraction or into HDL3. It also delivers cholesterol to the bile via the liver SRB1 pathway. An α-2 concentration value below 27 mg/dl is associated with increased heart disease risk in men and an α-2 blood concentration value below 34 mg/dl is associated with increased heart disease risk in women. An α-2 concentration value between 27 mg/dl and 34 mg/dl in men, and between 34 mg/dl and 45mg/dl in women, is considered borderline. An α-2 concentration value above 34 mg/dl in men and above 45 mg/dl in women is considered optimal.

The term "α-3 HDL particle (α-3)," as used herein, refers to a medium/small size particle containing apoA-I and apoA-II molecules, and probably is the smallest spherical HDL particle. Separated by other methods α-3 is part of the small HDL or HDL3a-b. An α-3 concentration value above 30mg/dl is associated with increased heart disease risk in both men and women. An α-3 concentration between 13.5 mg/dl and 30 mg/dl in both men and women is considered borderline. An α-3 concentration value below 13.5 mg/dl in both men and women is considered optimal.

The term "-4 HDL particle (α-4)," as used herein, refers to a small size particle containing apoA-I molecules, some phospholipids (PL) and free cholesterol. Separated by other methods α-4 is part of the small HDL or HDL3c. An α-4 concentration value above 25 mg/dl is associated with increased heart disease risk in both men and women. An α-4 concentration between 17 mg/dl and 25 mg/dl in both men and women is considered borderline. An α-4 blood concentration value below 13.5 mg/dl in both men and women is considered optimal.

The term "preα-mobility HDL particles (preα-)," as used herein, refers to HDL particles with similar sizes to the adjacent α-mobility particles, but with a different chemical composition. So far separation of preα-mobility particles can be obtained only by non-denaturing two-dimensional gel electrophoresis (ndPAGE).

The term "preβ-1 HDL particle (preβ-1)," as used herein, refers to an important HDL particle for predicting heart disease. So far only ndPAGE can separate the preβ-1 particles. This particle is the smallest among the apoA-I-containing HDL particles, and it contains 2 apoA-I and about 8-10 PL molecules. The preβ-1 HDL particle picks up cholesterol from the artery wall via the ATP-binding cassette protein 1 (ABCA1) pathway. An increased level marks an inadequate HDL metabolism and is associated with increased risk for CVD. A preβ-1 blood concentration value above 25 mg/dl is associated with increased heart disease risk in both men and women, while a preβ-1 blood concentration value between 17 mg/dl and 25 mg/dl in both men and women is considered borderline. A preβ-1 blood concentration value below 17 mg/dl in both men and women is considered optimal.

The term "preβ-2 HDL particle (preβ-2)," as used herein, refers to an HDL particle for predicting heart disease. This is a large but lipid-poor HDL particle. There are currently no methods other than ndPAGE that are able to separate these particles. Currently, there is no definition for the optimal preβ-2 blood concentration value.

The term "core coronary-risk panel" as used herein, refers to measurements on low-density lipoprotein cholesterol (LDL-C), apolipoprotein B (apo-B), high-density lipoprotein cholesterol (HDL-C), apolipoprotein A-I (apoA-I), HDL-map, C reactive protein (CRP), and lipoprotein associated phospholipase A2 (LpPLA2).

The term "coronary artery stenosis" as used herein, refers to a narrowing or constriction of the inner surface (lumen) of the coronary artery, usually caused by atherosclerosis.

The terms "correlate", "correlating" and "correlation" refer to identifying HDL particles within an HDL subpopulation, quantitating their level or concentration within a sample or subject, and comparing their level or concentration to a reference level or concentration. A "correlation" is established when a subject's level or concentration of a particular HDL particle is compared to a reference value, or range of values, and determined to be above or below that value, or within that range of values.

The term "CAP," as used herein, refers to calcified plaques in the artery wall. Voxels between the outer vessel wall boundary and the luminal boundary, and having attenuation values 150 Hounsfield Units (HU) or greater, are classified as CAP.

The term "cardiovascular disease (CVD)" refers to the group of diseases that affect the cardiovascular system, primarily the heart and blood vessels, including arteries and veins.

The term "high density lipoprotein (HDL) cholesterol (HDL-C)," as used herein, refers to the cholesterol in the "good particles" measured in plasma, after the removal of apo-B containing lipoproteins (very low density lipoprotein cholesterol and low density lipoprotein particles). High blood concentration values of HDL-C, above 60 mg/dl, protect against heart disease, a HDL-C blood concentration value between 40 and 60 mg/dl is considered borderline, while a low HDL-C blood concentration value, below 40 mg/dl in men and below 50 mg/dl in women, is associated with an increased heart disease risk.

The term "HDL subpopulation analysis by non-denaturing two-dimensional gel electrophoresis" or "two-dimensional gel electrophoresis," as used herein, refers to a technology that measures different HDL particles by directly separating the particles by electrophoretic charge and size, and then measuring the amount of apolipoprotein A-I in each particle by image analysis. This test not only measures the small HDL particles that pick up cholesterol from the artery wall, but also measures the intermediate particles, and the large HDL particles that deliver cholesterol to the liver. The HDL subpopulation analysis helps provide precise information about a person's heart disease risk that is qualitatively and quantitatively superior to the information available from traditional CVD-risk measurements. Also, measuring these particles provides invaluable information about how well a therapy-dietary and/or with medication(s)-is working for a patient.

By "HDL particle" is meant the various molecules that comprise an HDL subpopulation. For example, HDL particles that comprise the alpha subpopulation include, but are not limited to, α1, α2, α3, and α1-4; HDL particles that comprise the pre-alpha subpopulation include, but are not limited to, pre-α1, pre-α2, pre-α3, and pre-α1-4; and HDL particles that comprise the pre-beta subpopulation include, but are not limited to, pre-beta1 and pre-beta 2. The term encompasses not only the core molecules of a subpopulation (e.g. α1-4), but also all associated molecules including, for example, free cholesterol, phospholipids, cholesterol ester, triglycerides, and the like.

As used herein, the term "HDL population" denotes the totality of HDL subpopulations and/or particles that make up a particular HDL fraction of whole blood. For example, an HDL population includes, but is not limited to, the alphal-4 (α1-4), pre-alpha1-4 (pre-α1-4), and pre-beta1-2 (pre-β1-2) subpopulations, as well as the respective HDL particles within each subpopulation.

By "HDL subpopulation" is meant the various HDL particles that make up a HDL population. For example, an HDL subpopulation includes, but is not limited to, an alpha1-4 (α1-4) subpopulation, a pre-alpha1-4 (pre-α1-4) subpopulation, and/or a pre-beta1-2 (pre-β1-2) subpopulation.

By "HDL subpopulation profile" is meant the distribution and quantitation of HDL particles within the subpopulation as determined by either lipid or apolipoprotein contents using various methods including, but not limited to, ultracentrifugation, nuclear magnetic resonance analyses, size exclusion chromatography, and gel electrophoresis.

By "increased risk" is meant an HDL population or subpopulation from a sample or subject in which at least one HDL particle is present at a level or concentration higher than, or lower than, a reference value, thereby indicating that the sample or subject has a statistically significant likelihood of having soft plaques and/or developing CVD relative to a normal sample or subject.

By "increased risk profile value" is meant the level or concentration of HDL particles within an HDL population or subpopulation that indicates a statistically significant likelihood of having soft plaques and/or developing CVD relative to a normal sample or subject. For example, an increased risk profile value for an HDL population includes, but is not limited to, an α-1 value of <14 and <20 mg/dl, an α-2 value of <27 and <34mg/dl, an α-3 value of >30 and >30, and an α-4 value of >25 and >25 mg/dl, an a preβ-1 value of >25 and >25 mg/dl in men and women, respectively.

By "borderline risk" is meant an HDL population or subpopulation from a sample or subject in which at least one HDL particle is present at a level or concentration within a range of two reference values, thereby indicating that the sample or subject has a statistically significant likelihood of being predisposed to having soft plaques and/or developing CVD relative to a normal sample or subject.

By "borderline risk profile value" is meant the range of levels or concentrations of HDL particles within an HDL population or subpopulation that indicates a statistically significant likelihood of being predisposed to having soft plaques and/or developing CVD relative to a normal sample or subject. For example, a borderline risk profile value for an HDL population includes, but is not limited to, an α-1 value between 14-20 mg/dl and 20-30 mg/dl, an α-2 value between 27-34 mg/dl and 34-45 mg/dl, an α-3 value between 13.5-30 mg/dl and 13.5-30 mg/dl, and an α-4 value between 17-25 mg/dl and 17-25 mg/dl in men and women, respectively.

By "low risk" is meant an HDL population or subpopulation from a sample or subject in which at least one HDL particle is present at a level or concentration higher than, or lower than, a reference value, thereby indicating that the sample or subject does not have a statistically significant likelihood of having soft plaques and/or developing CVD relative to a normal sample or subject.

By "low risk profile value" is meant the level or concentration of HDL particles within an HDL population or subpopulation that indicates a statistically significant likelihood of having soft plaques and/or developing CVD relative to a normal sample or subject. For example, a low risk profile value for an HDL population includes, but is not limited to, an α-1 value of >20 and >30 mg/dl , an α-2 value of >34 and >45 mg/dl , an α-3 value of <13.5 and < 13.5 mg/dl , and an α-4 value of <25 and < 25 mg/dl in men and women, respectively.

By "suboptimal HDL subpopulation profile" is meant the distribution of HDL particles within the subpopulation having a significant deviation from that in gender and age-matched healthy subjects. Subjects with suboptimal HDL subpopulation profile usually have lower than normal levels of the large, lipid-rich HDL subclass (large HDL, or HDL2 or HDL2β, or alpha-1) and have higher than normal levels of the small, lipid-poor HDL subclasses (small HDL, or HDL3, or HDL3, HDL3b, HDL3c, or α-3 and 4, pre-α-3-and 4, and pre-β-1).

The term "HD-NCP," as used herein, refers to high density non-calcified plaque in the artery wall. Voxels between the outer vessel wall boundary and the luminal boundary, and having attenuation values of 30 to 149 HU, are classified as HD-NCP.

The term "LD-NCP," as used herein, refers to low density non-calcified plaque in the artery wall. Voxels between the outer vessel wall boundary and the luminal boundary, and having attenuation values of -100 to +30 HU, are classified as LD-NCP.

The term "NCP", as used herein, refers to non-calcified plaque in the artery wall. NCP is the sum of LD-NCP and HD-NCP.

The term "MLA", as used herein, refers to minimum lumen area of an artery calculated as mm2.

The term "%AS", as used herein, refers to the area of plaques determined as the difference between the calculated and actually determined lumen area.

The term "%DS", as used herein, refers to the area of plaques determined as the difference between the calculated and actually determined lumen diameter.

The term "MLD", as used herein, refers to minimum lumen diameter of an artery. Minimum lumen diameter is measured as the cross-section of the artery having the smallest size, and corresponds to the minimum voxel number between the inner wall of the measured segment of the coronary artery.

The term "MD-CT", as used herein, refers to multi-detector computed tomography. MD-CT technology uses an X-ray tube to generate energy through the body. A rotating gantry carries the X-ray tube, and rows of multiple detectors on the opposite side of the patient detect the attenuation of the X-rays as the X-rays penetrate the body. Images are reconstructed in a 3-dimensional fashion based on backprojection algorithms.

The term "IVUS", as used herein, refers to intravascular ultrasound. Intravascular ultrasound is a medical imaging methodology that uses a specially designed catheter with a miniaturized ultrasound probe attached to the distal end of the catheter. The proximal end of the catheter is attached to computerized ultrasound equipment. It allows the application of ultrasound technology to see from inside blood vessels out through the surrounding blood column, visualizing the endothelium (inner wall) of blood vessels in living individuals.

The term "vulnerable plaque", as used herein, refers to low density non-calcified plaque (LD-NCP) and high density non-calcified plaque (HD-NCP). LD-NCP is a collection of white blood cells (primarily macrophages) and lipids (including cholesterol) in the wall of an artery that is particularly unstable, and prone to produce sudden heart attack or stroke. HD-NCP has a thin fibrous cap and a large, soft lipid pool underlying the cap. These characteristics, in combination with normal hemodynamic pulsating expansion during systole and elastic recoil contraction during diastole, help create a high mechanical stress zone on the fibrous cap that is prone to rupture. Generally a non-calcified plaque becomes vulnerable if it grows more rapidly and has a thin cover separating it from the bloodstream inside the arterial lumen. Tearing of the cover is called plaque rupture. Repeated atheroma rupture and healing is one of the mechanisms, perhaps the dominant one, which creates artery stenosis.

As used herein, the singular forms "a", "an", and "the" include plural forms unless the context clearly dictates otherwise.

Unless specifically stated or obvious from context, as used herein, the term "about" is understood as within a range of normal tolerance in the art, for example within 2 standard deviations of the mean. The term "about" can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

As used herein, the terms "comprises," "comprising," "containing," "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

The term "including" is used herein to mean, and is used interchangeably with, the phrase "including, but not limited to."

Unless specifically stated, or obvious from context, the term "or" is understood to be inclusive as used herein.

By "agent" is meant any small molecule chemical compound, antibody, nucleic acid molecule, or polypeptide, or fragments thereof.

By "ameliorate" is meant decrease, suppress, attenuate, diminish, arrest, or stabilize the development or progression of a disease.

By "analog" is meant a molecule that is not identical, but has analogous functional or structural features. For example, a polypeptide analog retains the biological activity of a corresponding naturally-occurring polypeptide, while having certain biochemical modifications that enhance the analog's function relative to a naturally occurring polypeptide. Such biochemical modifications could increase the analog's protease resistance, membrane permeability, or half-life, without altering, for example, ligand binding. An analog may include an unnatural amino acid.

"Detect" refers to identifying the presence, absence, or amount of the lipoprotein to be detected.

By "detectable label" is meant a composition that when linked to a molecule of interest renders the latter detectable, via spectroscopic, photochemical, biochemical, immunochemical, or chemical means. For example, useful labels include radioactive isotopes, magnetic beads, metallic beads, colloidal particles, fluorescent dyes, electron-dense reagents, enzymes (for example, as commonly used in an ELISA), biotin, digoxigenin, or haptens.

By "disease" is meant any condition or disorder that damages or interferes with the normal function of a cell, tissue, or organ. Examples of diseases include coronary heart disease, atherosclerosis, *etc.*

By "effective amount" is meant the amount required to ameliorate the symptoms of a disease relative to an untreated patient. The effective amount of active compound(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

By "marker" is meant any molecule having an alteration in expression level or activity that is associated with its function or a disease or disorder.

By "reduces" is meant a negative alteration of at least 10%, 25%, 50%, 75%, or 100%.

By "reference" is meant a standard or control condition.

By "specifically binds" is meant a compound that recognizes and binds a compound of the invention, but which does not substantially recognize and bind other molecules in a sample, for example, a biological sample, which naturally includes a polypeptide of the invention.

By "subject" is meant a mammal, including, but not limited to, a human or non-human mammal, such as a bovine, equine, canine, ovine, or feline.

Ranges provided herein are understood to be shorthand for all of the values within the range. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

As used herein, the terms "treat," "treating," "treatment," and the like refer to reducing or ameliorating a disorder and/or symptoms associated therewith. It will be appreciated that, although not precluded, treating a disease, disorder, or condition does not require that the disease, disorder, condition, or symptoms associated therewith be completely eliminated.

### BRIEF DESCRIPTION OF THE DRAWINGS

The invention may be more completely understood in consideration of the following detailed description of various embodiments of the invention in connection with the accompanying figures, in which:
Figure 1A and 1B are graphs that illustrate the regression analyses on the correlation between low density non-calcified plaque percentile and LDL-C level, or HDL-C level, respectively.
Figure 2A-2D are graphs that illustrate correlations between plaque volume and HDL-C level, α-1 level, α-3 level, and α-4 level, respectively.
Figure 3A-3D are graphs that illustrate correlations between LD-NCP volume and HDL-C level, TG level, α-1 level, and α-3 level, respectively.

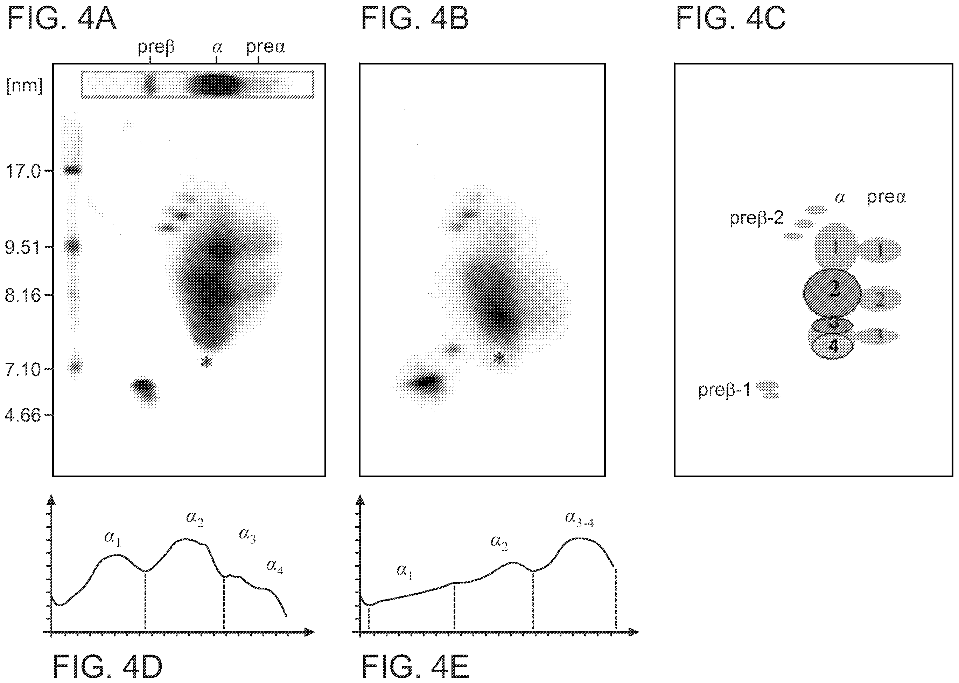

Figure 4A and 4B show immunoblots of HDL subpopulations, Figure 4C shows a schematic of HDL subpopulations, and Figures 4D and 4E shows charts of alpha subpopulation concentrations corresponding to the immunoblots of Figures 4A and 4B, respectively. Figure 4A and 4B illustrate typical results of the HDL subpopulation analysis assessed by two-dimensional gel electrophoresis, immuno-localization, image analysis, for a healthy control subject and a subject with CHD, respectively. HDL particles are separated from whole plasma by agarose-gel electrophoresis by charge into pre-β, α, and pre-α mobility subfractions (shown in the horizontal dimension), and are further separated by size in the vertical dimension (from large on top to small on the bottom of the gel). Figure 4C shows the schematic representation of HDL particles containing only apoA-I (LpA-I) (shown as light grey) and HDL particles containing both apoA-I and apoA-II (LpA-I:A-II) (shown as dark grey), as well as the positions of all apoA-I containing HDL particles. The "*" indicates the position of albumin, a 67kD plasma protein, that marks the α-front. Figure 4D is a chart that represents the integration of the α-mobility HDL particles, used to define the positions of the individual α-mobility particles in a normal subject, while Figure 4E represents a similar chart for a subject at increased risk of CVD. Figures 4A-4E demonstrate the effectiveness of two-dimensional gel electrophoresis as applied to the present invention.
Figure 5A-5D are tables summarizing the stepwise R-square analyses of associations between plaque characteristics and the used surrogate markers (HDL-C, apoA-I, HDL subpopulations, LDL-C, apoB, CRP, and LpPLA2).
Figure 6 is a table that summarizes data of a prospective multivariate analysis on the associations between changes in plaque composition and minimum lumen diameter and changes in parameters measured in lipoproteins, according to an aspect of the present invention.
Figures 7A and 7B depict how the individual HDL subclasses, as well as their respective HDL particles, are delineated for quantitation. The image quant analysis program calculates the signal volume in each area (Figure 7A, outlined regions), and then calculates and deducts the background (Figure 7A, rectangle) from the result. It also calculates the percent distribution of the signal under the designated areas. Figure 7B shows a cartoon schematic of the subpopulations and particles of Figure 7A.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention as defined by the appended claims.

### DETAILED DESCRIPTION OF THE INVENTION

The present invention pertains to a method for determining a subjects risk of plaque accumulation within a blood cell according to claim 1 or claim 6. In particular, the invention features methods that are useful for identifying a subject as having, or having a propensity to develop, cardiovascular disease (e.g., atherosclerosis).

The invention is based, at least in part, on the discovery that a core coronary risk panel of CVD markers is useful for identifying subjects at increased risk for CVD. Notably, detection and analysis of alpha, pre-alpha, and pre-beta HDL subpopulations is useful for assessing CVD because specific HDL subpopulation profiles were experimentally correlated with subjects that either displayed, or did not display, manifestations of CVD, such as calcified or non-calcified plaque. Moreover,
analysis of a large population of subjects revealed that specific HDL subpopulation profiles were useful for identifying subjects at increased risk, borderline risk, or low risk of developing CVD.

Additionally, the specific HDL subpopulation profiles were also useful for assessing stenosis, plaque vulnerability, and plaque volume. There is also described methods involving estimating cardiovascular risk posed by high plaque volumes, especially high volumes of LD-NCP in arteries, which involves determining coronary artery plaque composition/stability utilizing LDL-C, apoB, and HDL-C, apoA-I as well as the HDL subpopulation profile as determined by non-denaturing two-dimensional gel electrophoresis and image analysis. Blood testing is conducted to determine the levels of selected lipids, lipoproteins, apolipoproteins, enzymes, and transfer proteins and inflammatory markers in plasma while MD-CT, and IVUS are utilized for measuring and quantifying the various parameters necessary for the evaluation.

Atherosclerosis is a progressive disease and the different types of plaques are metabolically different. Low-density and high-density non calcified plaques are metabolically active and can shrink or progress into HD-NCP and calcified plaque forms, respectively, depending on the conditions. In contrast, calcified plaques are not metabolically active and cannot change composition or geometry, although, adjacent HD-NCP can be calcified and fuse with established CAP resulting in size increase.

ApoA-I levels in HDL subclasses are significantly associated with relative and total plaque volume, as well as with plaque geometry and composition in coronary arteries, as determined by NUS and MD-CTA. Therefore, a measurement of the core risk panel from blood samples is useful for estimating the severity of coronary heart disease, plaque volume, stenosis and plaque vulnerability.

The various core coronary-risk markers are associated with different stages of the disease. HDL-associated parameters, HDL-C, apoA-I, and HDL subpopulation profile (the HDL-map), are mainly associated with the early and intermediate forms of the disease like the LD-NCP and HD-NCP measures. LDL-associated parameters (LDL-C and apoB) and inflammatory markers (CRP and LpPLA₂) are mainly associated with the later form of the disease like HD-NCP and CAP measures and coronary artery stenosis like MLD, MLA, %AS, and %DS. Therefore, the core coronary-risk panel measured from the patients' plasma not only can estimate the
severity of disease burden, but also can give information about the chronic and the acute phases of the disease. Analyses indicated that measurements of LDL-C, apoB, and the inflammatory markers are good metrics for overall plaque volume and coronary stenosis, while HDL-subpopulation profile (HDL-map) is useful for estimating the volume/percentile of low-density and high-density NCP in the coronary arteries. The inflammatory markers, CRP and LpPLA2 can be used as metrics for the speed of conversion from LD-NCP into HD-NCP and HD-NCP into CAP. Moreover, repeated measurements of the core coronary-risk panel can provide estimation of changes in disease burden or efficacy of the applied treatment.

There is described a method for identifying a subject with increased risk of cardiovascular disease (CVD), a method of monitoring a subject with increased risk of CVD, a method of identifying a subject with increased risk of stenosis or non-calcified plaque formation, and a method of treating a subject having CVD.

The inventive methods involve providing an HDL subpopulation profile by separating and quantitating the ApoA-T content of HDL particles from a whole blood or plasma sample, and correlating this profile with reference profile values indicative of a risk for CVD, a borderline risk for CVD, or a low risk of CVD. Reference profile values may include absolute values of specific members of the HDL subpopulation (e.g. α-1, α-2, α-3, α-4, pre-β-1, and the like) and/or ratios of values of such specific members of the HDL subpopulation (e.g. α-1/α-2, α-1/α-3, α-1/α-4, and the like). Additionally, reference profile values may be fmiher correlated with at least one additional CVD risk factor such as, but not limited to, age, gender, hypertension, smoking, diabetes, family history of CVD, high LDL-C, and/or low HDL-C plaque.

A subject, or group of subjects, is selected and blood samples are collected at time zero (the 1st blood sample collection). Subsequent blood samples may be collected at time one, time two, time three, and the like, as necessary to identify, monitor, and/or treat the CVD. Sample collection periods can be any period required to identify, monitor, and/or treat the CVD, such as, but not limited to, every month, six months, year, 2 years, 5 years, and the like.

For the selected subject, or group of subjects, HDL subpopulation profiles are provided from blood samples taken at each time point. HDL subpopulation profiles may be determined by, for example, separating the subpopulation by non-denaturing,
two dimensional gel electrophoresis (e.g. ndPAGE), followed by immunoblotting and image analysis. Although ndPAGE has the highest specificity and resolution other methods like non-denaturing, one dimensional gel electrophoresis, ultracentrifugal separation, size exclusion chromatography and NMR are also used for subfractionating HDL. The image analysis allows quantitation of specific members of the subpopulation, and generation of their specific values. For example, the specific members can include proteins from the α subpopulation (*e.g.* α-1, α-2, α-3, and α-4), the pre-α subpopulation (*e.g.* pre-α-1, pre-α-2, and pre-α-3), and/or the pre-β subpopulation (*e.g.* pre-β-1 and pre-β-2), without limitation.

These profiles are then correlated with *(i.e.* compared to) reference values in order to assess the subject's risk for CVD. Such HDL subpopulation profiles may be based on the absolute value of specific members of the HDL subpopulation. It is also contemplated that such subpopulation profiles may be based on ratios of specific members of the HDL subpopulation. Reference values have been obtained from analysis of CVD related data from large population studies (Metabolic and functional relevance of HDL subspecies. Asztalos BF et al. Curr Opin Lipidol. 2011 Jun;22(3):176-85.). For example, an increased risk profile for a subject may include the instance where the α subpopulation comprises values of α-1 (13.5 and 19.1 mg/dl), α-2 (27.0 and 33.8 mg/dl), α-3 (13.5 and 10.0 mg/dl), and α-4 (13.5 and 10.0 mg/dl), in men (see, *e.g.,* Table 1) and women (see, *e.g.,* Table 2), respectively Similar profiles may be generated from subpopulation profile values for borderline and low risk subpopulations. It is worth noting that these numbers are population-based cut points, but these are continuous variables and the larger the differences from normal (lower in case of α-1 and α-2 and higher in case of α-3 and α-4) the higher the risk. For example, a male subject with an α-1 value of 5 mg/dl would be considered at higher increased risk than a male subject with an α-1 value of 13 mg/dl.

**Table 1. HDL subpopulation reference values for men**

| Risk | α-1 (mg/dl) | α-2 (mg/dl) | α-3 (mg/dl) | α-4 (mg/dl) | preβ-1 (mg/dl) |
|---|---|---|---|---|---|
| Increased | <14 | <27 | >30 | >25 | >25 |
| Borderline | 14-20 | 27-34 | 13.5-30 | 17-25 | 17-25 |
| Low | >20 | >34 | <13.5 | <25 | <17 |

**Table 2. HDL subpopulation reference values for women**

| Risk | α-1 (mg/dl) | α-2 (mg/dl) | α-3 (mg/dl) | α-4 (mg/dl) | preβ-1 (mg/dl) |
|---|---|---|---|---|---|
| Increased | <20 | <34 | >30 | >25 | >25 |
| Borderline | 20-30 | 34-45 | 13.5-30 | 17-25 | 17-25 |
| Low | >30 | >45 | <13.5 | <25 | <17 |

Correlation of subject HDL subpopulation profiles may be made by directly comparing the value of specific members of the HDL subpopulation of the subject to one or more of the reference values (e.g. high risk profile values, borderline risk profile values, and low risk profile values). Alternatively, correlation of subject HDL subpopulation profiles may be made by comparing ratios of specific members of the HDL subpopulation of the subject to one or more of the reference ratio values.

It is further contemplated that these correlations may be strengthened by further correlating the HDL subpopulation profiles with at least one additional CVD risk factor. For example, such additional factors may include, but are not limited to, the following: age, gender, hypertension, smoking, diabetes, family history of CVD, high LDL-C, and CRP and LpPLA2. It is contemplated that such additional correlation analysis may use two, three, four, five, or more of the additional factors.

The correlation will allow determination of whether a subject, or a group of subjects, has an HDL subpopulation profile that is indicative of having a risk for CVD, having a borderline risk for CVD, of having a low risk for CVD. Additionally, such HDL subpopulation profiles will allow a subject to be monitored to determine whether, for example, intervention measures have a positive or negative effect on the subject's HDL subpopulation profile, thereby indicating whether the subject's risk profile has improved, diminished, or stayed the same.

### Example 1: Correlation of plaque formation with biomarker concentration

Sixty patients having established coronary artery disease (CAD) were recruited for a prospective study. These patients underwent coronary MD-CTA and IVUS measurements. Blood samples from the patients were collected at baseline for biomarker analysis. Fifty-six patients returned 12 months later for a repeat IVUS and MD-CTA analysis and for biomarker analysis.

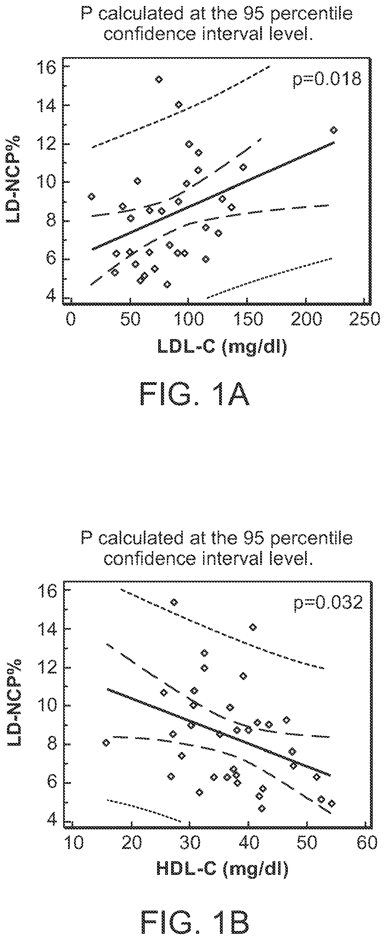

As shown in Figure 1A and 1B, increases in LDL-C concentration in the blood were strongly correlated with increased levels of LD-NCP. Conversely, increases in HDL-C concentration were associated with decreases in LD-NCP.

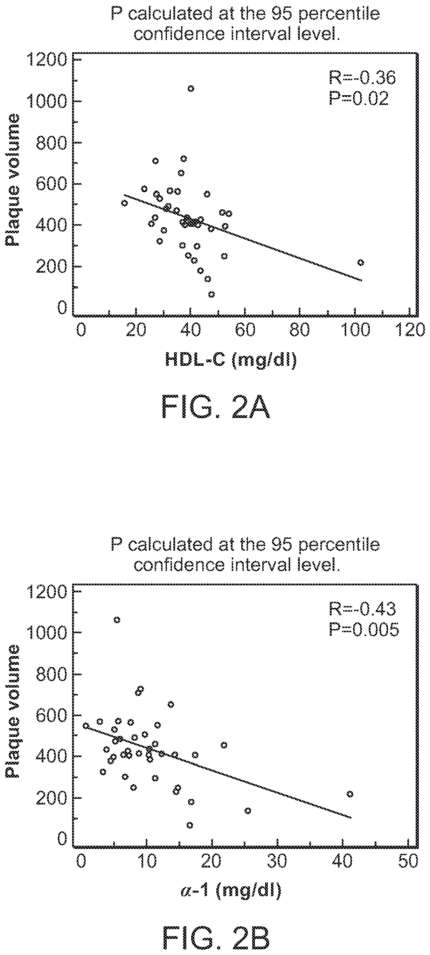

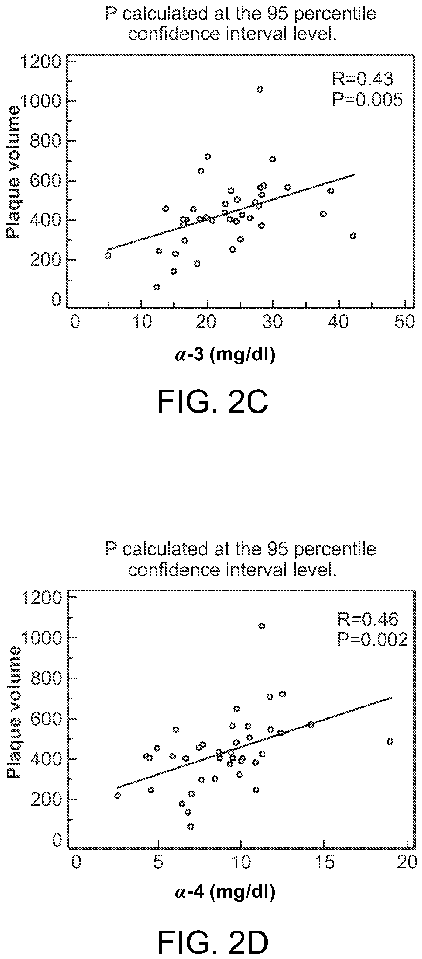

Plaque volume was then correlated with HDL-C, α-1, α-3, and α-4. As shown in Figure 2A and 2B, plaque volume decreased with increasing concentrations of HDL-C and α-1. In contrast, plaque volume was found to increase with increasing concentrations of α-3 and α-4.

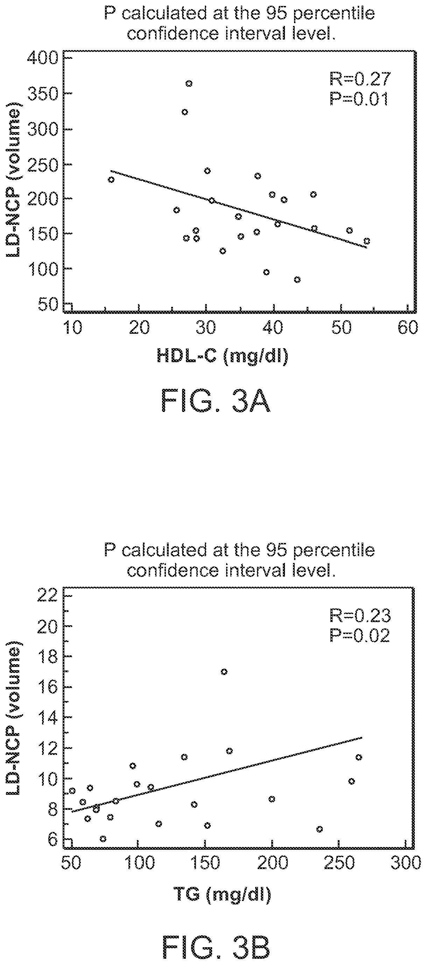

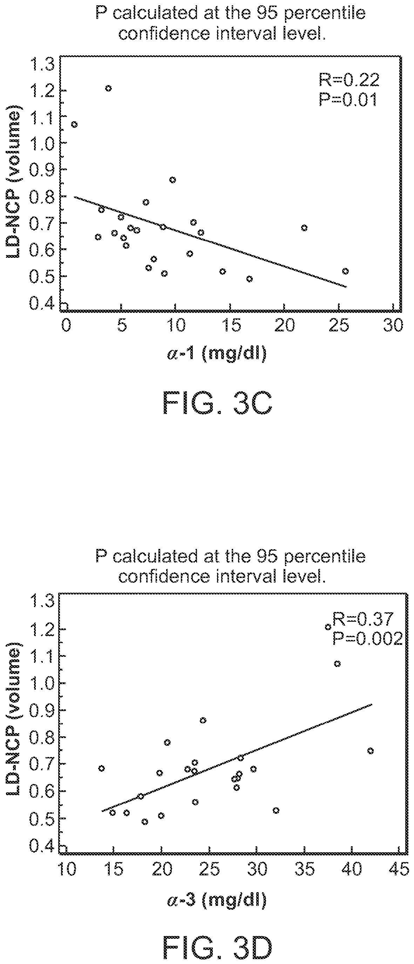

LD-NCP volume was then correlated with HDL-C, TG, α-1, and α-3. As shown in Figure 3A, LD-NCP volume decreases as HDL-C concentration increases. Conversely, as shown in Figure 3B, LD-NCP volume increases as TG concentration increases. LD-NCP volume decreases as α-1 volume increases, while LD-NCP volume increase with increasing concentration of α-3, as shown in Figure 3C and 3D, respectively.

Analysis of HDL subpopulations from normal patients and patients with CVD is shown in Figures 4A and 4D and Figures 4B and 4E, respectively. In the patient with CVD, there was a pronounced shift in the distribution of the alpha subpopulation comprising low levels of α-1 and α-2, and high levels of α-3 and α-4 (Figure 4E), relative to the normal patient in which the α-1 and α-2 level are high and the α-3 and α-4 levels are relatively low.

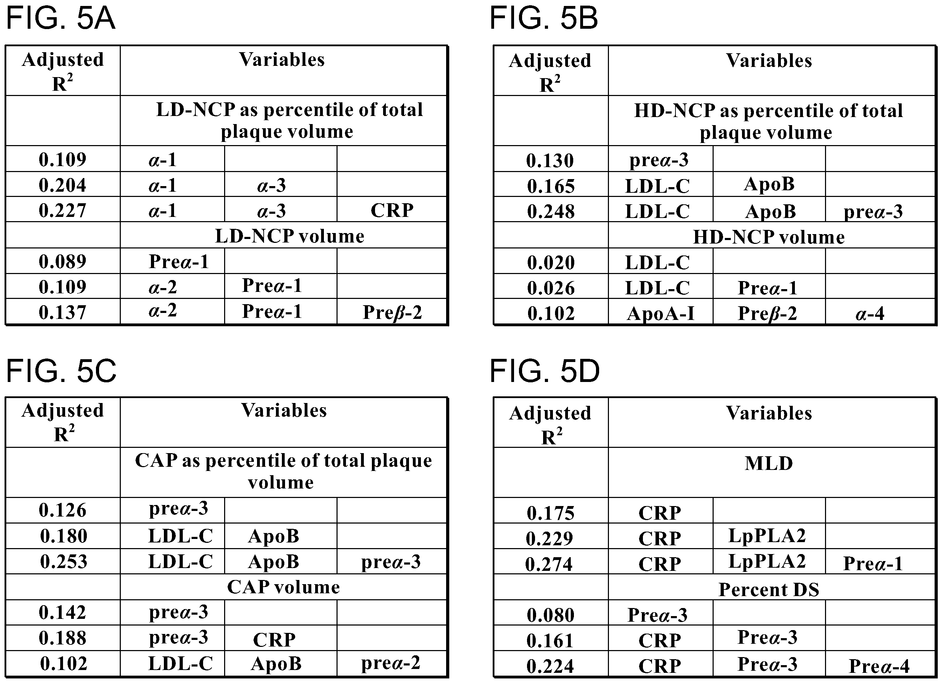

The stepwise R-square analyses of associations between plaque characteristics and the surrogate markers HDL-C, apoA-I, HDL subpopulations, LDL-C, apoB, CRP, and LpPLA2 is summarized in Figures 5A-5D.

### Example 2: Multivariate analysis of subjects with cardiovascular disease

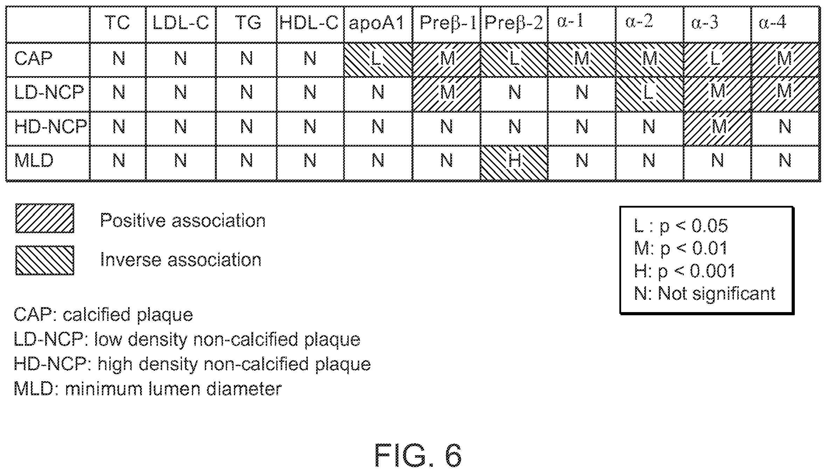

A stepwise selection method including from 1 up to 3 dependent-parameters indicated that the association (adjusted R-square) with disease status (plaque parameters) improved with increasing number of variables in the model. These analyses are illustrated in Figure 6.

The analysis may be summarized as follows. LD-NCP parameters showed the highest associations with HDL-map-related parameters. HD-NCP parameters showed associations with both HDL-related and LDL-related parameters. CAP parameters showed associations with HDL, LDL and inflammation-related parameters. MLD and %DS were best associated with the two inflammatory markers and HDL related parameters.

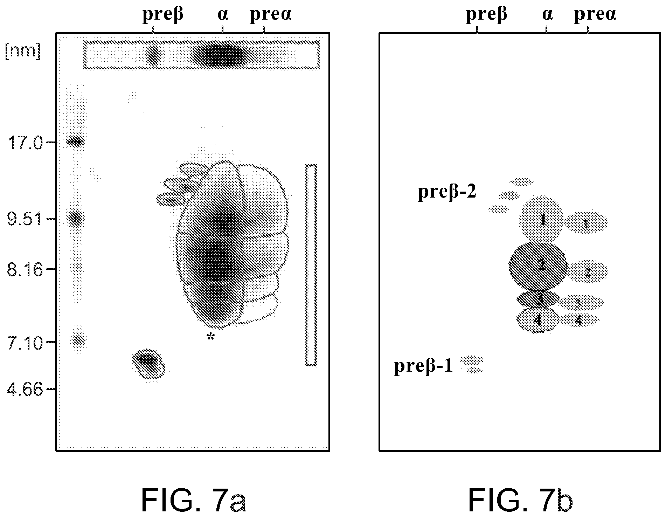

A multivariate analysis, with respect to the associations between coronary-artery parameters and HDL parameters, was conducted utilizing the 60 patients' data. Changes in plaque characteristics were associated with changes in the lipoprotein measurements (Figure 7). In a stepwise fashion each of the core coronary-risk panel variables was treated as dependent parameter and adjusted for the rest of the variables. Significant association was based on p<0.05 at the 95% confidence interval level. As summarized in Figure 7, the analysis revealed the following correlations:
1. High apoA-I level is associated with a lower volume of CAP, however, this did not translate into plaque geometry;
2. High prep-1 HDL particle level is associated with more CAP, which can cause more stenosis, and more LD-NCP volume, which represents a more vulnerable phenotype; consequently, high preP-1 HDL particle level indicates increased risk;
3. High preβ-2 HDL particle level is associated with less CAP and less stenosis (or large MLD; it is also associated with a shift from LD-NCP to HD-NCP (and higher MLD), and is therefore a protective particle;
4. The α-1 particle level was associated with a shift from LD-NCP to CAP, which is a shift that tends to make plaque more stable; consequently, this particle is also protective;
5. High preα-1 particle level is associated with less total plaque volume (CAP, LD-NCP, and HD-NCP), and this particle is therefore protective;
6. The α-2 particle level is associated with a shift from LD-NCP to CAP-a shift that makes plaque more stable-and is therefore also protective;
7. The preα-2 particle level is associated with less total plaque volume (CAP, LD-NCP, and HD-NCP), and is therefore also protective;
8. The α-3 particle level is associated with a shift from CAP to non-calcified plaques, such as LD-NCP and HD-NCP, which tends to make plaques less stable; therefore, these particles are harmful;
9. The α-4 particle level is associated with increased LD-NCP and CAP, which makes plaque less stable; therefore these particles are also harmful;
10. High HDL-C level is associated with less of the relative (%) and absolute level (volume) of LD-NCP, and therefore contributes to plaques with less vulnerable phenotype; consequently, it is atheroprotective;
11. High LDL-C level is associated with more of the relative (%) and absolute level (volume) of LD-NCP; consequently, these plaques have a more vulnerable phenotype, so these particles are harmful.

A brief description of the method for analyzing the apoA-I-containing HDL subpopulation by non-denaturing two-dimensional gel electrophoresis, immunoblotting, and image analysis: Four µL of plasma was applied and electrophoresed in a vertical slab agarose gel (0.7% LE) in the first dimension at 250 V until the α-mobility front moved 3.5 cm from the origin. The agarose gel was sliced and the stripes were applied to the top of 3-35% non-denaturing concave gradient polyacrylamide gels. In the second dimension, gels were electrophoresed at 265 V for 21 hours at 10°C and then electro-transferred to nitrocellulose membranes at 31 V for 23 hours at 10°C. To achieve reproducibility, the uniformity of the concave gradient gels was maintained by controlling the shape of the gradient, the rate of polymerization, and the temperature. Protocols for electrophoreses and transfer were strictly followed.

ApoA-I was immunolocalized on the membrane with monospecific goat antihuman first and ¹²⁵I labeled secondary antibodies (immunopurified rabbit F(ab')2 fraction against goat IgG). The bound ¹²⁵I -labeled secondary antibody was quantified in a FluoroImager (Molecular Dynamics, CA).

Quantitative image analysis of the apoA-I-containing HDL subspecies revealed that preβ-1 and preβ-2 particles do not overlap with any other HDL particles, consequently, they are easily delineated. Designation of the α-mobility HDL subpopulations as based on integration curves of α-migrating HDL as shown in Figure 1A and 1B. Each peak area for α-1, α-2, α-3 and α-4 was delineated (Figure 7). Preα-mobility particles have the same size as their α-mobility counterparts. Finally 10 apoA-I-containing HDL subpopulations were encircled; signals were measured in each area, and used for calculating the percent distribution of the apoA-I-containing HDL subpopulations. Data are expressed as pixels linearly correlated with the disintegrations of the ¹²⁵I bound to the antigen-antibody complex/min. Samples were characterized by 1) their charge (preβ, α, and preα) based on their relative mobility to albumin and 2) their size, determined from the molecular weight standard (¹²⁵I-labeled Pharmacia high molecular weight standards) run simultaneously in the same gel. Results were calculated from duplicate separations. ApoA-I concentrations of the subpopulations were calculated by multiplying percentiles of distribution by plasma total apoA-I concentrations. For example if a person has a 150 mg/dl apoA-I and 10% α-1 the α-1 concentration is: 150*(10/100) =15 mg/dl.

As noted above, the present invention pertains to methods for evaluating coronary artery disease in an individual by evaluating plaque volume and composition/stability via the core coronary-risk panel including the HDL subpopulation analyses. The present invention should not be considered limited to the particular embodiments described above, but rather should be understood to cover all aspects of the invention as fairly set out in the appended claims.

## Claims

1. A method for determining a male subject's risk of plaque accumulation within a blood vessel, comprising:
separating Apolipoprotein A-1 (ApoA-I) containing high-density lipoprotein (HDL) particles from a plasma sample of the male subject to obtain HDL subpopulations, wherein the HDL subpopulations comprise alpha (α)-1 particles, α-2 particles, α-3 particles, and α-4 particles;
quantitating the particles of the HDL subpopulations to thereby provide an HDL subpopulation profile;
providing a reference HDL subpopulation profile associated with risk of plaque accumulation selected from the group consisting of an increased risk profile, a borderline risk profile, and a low risk profile, wherein an increased risk profile comprises an α-1 value of <14 mg/dl, an α-2 value of <27 mg/dl, an α-3 value of >30, and an α-4 value of >25 mg/dl, wherein a borderline risk profile comprises an α-1 value between 14-20 mg/dl, an α-2 value between 27-34 mg/dl, an α-3 value between 13.5-30 mg/dl, and an α-4 value between 17-25 mg/dl, and wherein a low risk profile comprises an α-1 value of >20, an α-2 value of >34, an α-3 value of <13.5, and an α-4 value of <25;
comparing said HDL subpopulation profile with the reference HDL subpopulation profile to thereby determine a risk of plaque accumulation in the male subject.

2. The method according to claim 1, wherein the HDL subpopulations further comprise pre-β-1 particles and the increased risk profile further comprises a pre-β-1 value of >25.

3. The method according to claim 1, wherein the HDL subpopulations further comprise pre-β-1 particles and the borderline risk profile further comprises a pre-β-1 value between 17 -25 mg/dl.

4. The method according to claim 1, wherein the HDL subpopulations further comprise pre-β-1 particles and the low risk profile value comprises a pre-β-1 value <17 mg/dl.

5. A method for determining a female subject's risk of plaque accumulation within a blood vessel, comprising:
separating ApoA-I containing HDL particles from a plasma sample of the female subject to obtain HDL subpopulations, wherein the HDL subpopulations comprise alpha (α)-1 particles, α-2 particles, α-3 particles, and α-4 particles;
quantitating the particles of the HDL subpopulations to thereby provide an HDL subpopulation profile;
providing a reference HDL subpopulation profile associated with risk of plaque accumulation selected from the group consisting of an increased risk profile, a borderline risk profile, and a low risk profile, wherein an increased risk profile comprises an α-1 value of <20 mg/dl, an α-2 value of <34 mg/dl, an α-3 value of >30, an α-4 value of>25 mg/dl, wherein a borderline risk profile comprises an α-1 value between 20-30 mg/dl, an α-2 value between 34-45 mg/dl, an α-3 value between 13.5-30 mg/dl, and an α-4 value between 17-25 mg/dl, and wherein a low risk profile comprises an α-1 value of>30, an α-2 value of>45, an α-3 value of <13.5, and an α-4 value of <25;
comparing said HDL subpopulation profile with the reference HDL subpopulation profile to thereby determine a risk of plaque accumulation in the female subject.

6. The method according to claim 5, wherein the HDL subpopulations further comprise pre-β-1 particles and the increased risk profile further comprises a pre-β-1 value of>25 mg/dl.

7. The method according to claim 5, wherein the HDL subpopulations further comprise pre-β-1 particles and the borderline risk profile further comprises a pre-β-1 value between 17 -25 mg/dl.

8. The method according to claim 5, wherein the HDL subpopulations further comprise pre-β-1 particles and the low risk profile value comprises a pre-β-1 value < 17 mg/dl.

## Patentansprüche

1. Verfahren zum Ermitteln eines Risikos einer Plaqueansammlung innerhalb eines Blutgefäßes eines männlichen Individuums, das umfasst:
Trennen von Apolilipoprotein A-1 (ApoA-I) haltigen Partikeln von Lipoprotein mit hoher Dichte (HDL) aus einer Plasmaprobe des männlichen Individuums, um HDL-Subpopulationen zu erhalten, wobei die HDL-Subpopulationen alpha-(α)-1-Partikel, α-2-Partikel, α-3-Partikel und α-4-Partikel umfassen;
Quantifizieren der Partikel der HDL-Subpopulationen, um damit ein HDL-Subpopulationsprofil bereitzustellen;
Bereitstellen eines Referenz-HDL-Subpopulationsprofils, das mit einem Risiko einer Plaqueansammlung assoziiert ist, ausgewählt aus der Gruppe, bestehend aus erhöhtem Risikoprofil, Grenzrisikoprofil und niedrigem Risikoprofil, wobei ein erhöhtes Risikoprofil einen α-1-Wert von < 14 mg/dl, einen α-2-Wert von < 27 mg/dl, einen α-3-Wert von > 30 und einen α-4-Wert von > 25 mg/dl umfasst, wobei ein Grenzrisikoprofil einen α-1-Wert zwischen 14 und 20 mg/dl, einen α-2-Wert zwischen 27 und 34 mg/dl, einen α-3-Wert zwischen 13,5 und 30 mg/dl und einen α-4-Wert zwischen 17 und 25 mg/dl umfasst und wobei ein niedriges Risikoprofil einen α-1-Wert von > 20, einen α-2-Wert von > 34, einen α-3-Wert von < 13,5 und einen α-4-Wert von < 25 umfasst;
Vergleichen des HDL-Subpopulationsprofils mit dem Referenz-HDL-Subpopulationsprofil, um damit ein Risiko einer Plaqueansammlung beim männlichen Individuum zu ermitteln.

2. Verfahren nach Anspruch 1, wobei die HDL-Subpopulationen ferner Vor-β-1-Partikel umfassen und das erhöhte Risikoprofil ferner einen Vor-β-1-Wert von > 25 umfasst.

3. Verfahren nach Anspruch 1, wobei die HDL-Subpopulationen ferner Vor-β-1-Partikel umfassen und das Grenzrisikoprofil ferner einen Vor-β-1-Wert zwischen 17 und 25 mg/dl umfasst.

4. Verfahren nach Anspruch 1, wobei die HDL-Subpopulationen ferner Vor-β-1-Partikel umfassen und das niedrige Risikoprofil ferner einen Vor-β-1-Wert von < 17 mg/dl umfasst.

5. Verfahren zum Ermitteln eines Risikos einer Plaqueansammlung innerhalb eines Blutgefäßes eines weiblichen Individuums, das umfasst:
Trennen von ApoA-I-haltigen HDL-Partikeln aus einer Plasmaprobe des weiblichen Individuums, um HDL-Subpopulationen zu erhalten, wobei die HDL-Subpopulationen alpha-(α)-1-Partikel, α-2-Partikel, α-3-Partikel und α-4-Partikel umfassen;
Quantifizieren der Partikel der HDL-Subpopulationen, um damit ein HDL-Subpopulationsprofil bereitzustellen;
Bereitstellen eines Referenz-HDL-Subpopulationsprofils, das mit einem Risiko einer Plaqueansammlung assoziiert ist, ausgewählt aus der Gruppe, bestehend aus erhöhtem Risikoprofil, Grenzrisikoprofil und niedrigem Risikoprofil, wobei ein erhöhtes Risikoprofil einen α-1-Wert von < 20 mg/dl, einen α-2-Wert von < 34 mg/dl, einen α-3-Wert von > 30 und einen α-4-Wert von > 25 mg/dl umfasst, wobei ein Grenzrisikoprofil einen α-1-Wert zwischen 20 und 30 mg/dl, einen α-2-Wert zwischen 34 und 45 mg/dl, einen α-3-Wert zwischen 13,5 und 30 mg/dl und einen α-4-Wert zwischen 17 und 25 mg/dl umfasst und wobei ein niedriges Risikoprofil einen α-1-Wert von > 30, einen α-2-Wert von > 45, einen α-3-Wert von < 13,5 und einen α-4-Wert von < 25 umfasst;
Vergleichen des HDL-Subpopulationsprofils mit dem Referenz-HDL-Subpopulationsprofil, um damit ein Risiko einer Plaqueansammlung beim weiblichen Individuum zu ermitteln.

6. Verfahren nach Anspruch 5, wobei die HDL-Subpopulationen ferner Vor-β-1-Partikel umfassen und das erhöhte Risikoprofil ferner einen Vor-β-1-Wert von > 25 umfasst.

7. Verfahren nach Anspruch 5, wobei die HDL-Subpopulationen ferner Vor-β-1-Partikel umfassen und das Grenzrisikoprofil ferner einen Vor-β-1-Wert zwischen 17 und 25 mg/dl umfasst.

8. Verfahren nach Anspruch 5, wobei die HDL-Subpopulationen ferner Vor-β-1-Partikel umfassen und das niedrige Risikoprofil ferner einen Vor-β-1-Wert von < 17 mg/dl umfasst.

## Revendications

1. Procédé pour déterminer un risque d'accumulation de plaque dans un vaisseau sanguin chez un sujet masculin, comprenant :
la séparation des particules de lipoprotéine de haute densité (HDL) contenant l'apolipoprotéine A-1 (ApoA-I) à partir d'un échantillon de plasma du sujet masculin afin d'obtenir des sous-populations de HDL, où les sous-populations de HDL comprennent des particules alpha (α)-1, des particules α-2, des particules α-3, et des particules α-4 ;
la quantification des particules des sous-populations de HDL pour ainsi obtenir un profil de sous-populations de HDL ;
la mise à disposition d'un profil de sous-populations de HDL de référence associé à un risque d'accumulation de plaque sélectionné dans le groupe consistant en un profil de risque accru, un profil de risque limite, et un profil de faible risque, où un profil de risque accru comprend une valeur α-1 < 14 mg/dl, une valeur α-2 < 27 mg/dl, une valeur α-3 > 30, et une valeur α-4 > 25 mg/dl, où un profil de risque limite comprend une valeur α-1 comprise entre 14-20 mg/dl, une valeur α-2 comprise entre 27-34 mg/dl, une valeur α-3 comprise entre 13,5-30 mg/dl, et une valeur α-4 comprise entre 17-25 mg/dl, et où un profil de faible risque comprend une valeur α-1 > 20, une valeur α-2 > 34, une valeur α-3 < 13,5, et une valeur α-4 < 25 ;
la comparaison dudit profil de sous-populations de HDL avec le profil de sous-populations de HDL de référence pour ainsi déterminer un risque d'accumulation de plaque chez le sujet masculin.

2. Procédé selon la revendication 1, dans lequel les sous-populations de HDL comprennent en outre des particules pré-β-1 et le profil de risque accru comprend en outre une valeur pré-β-1 > 25.

3. Procédé selon la revendication 1, dans lequel les sous-populations de HDL comprennent en outre des particules pré-β-1 et le profil de risque limite comprend en outre une valeur pré-β-1 comprise entre 17-25 mg/dl.

4. Procédé selon la revendication 1, dans lequel les sous-populations de HDL comprennent en outre des particules pré-β-1 et la valeur du profil de faible risque comprend une valeur pré-β-1 < 17 mg/dl.

5. Procédé pour déterminer un risque d'accumulation de plaque dans un vaisseau sanguin chez un sujet féminin, comprenant :
la séparation des particules de HDL contenant l'ApoA-I à partir d'un échantillon de plasma du sujet féminin afin d'obtenir des sous-populations de HDL, où les sous-populations de HDL comprennent des particules alpha (α)-1, des particules α-2, des particules α-3, et des particules α-4 ;
la quantification des particules des sous-populations de HDL pour ainsi obtenir un profil de sous-populations de HDL ;
la mise à disposition d'un profil de sous-populations de HDL de référence associé à un risque d'accumulation de plaque sélectionné dans le groupe consistant en un profil de risque accru, un profil de risque limite, et un profil de faible risque, où un profil de risque accru comprend une valeur α-1 < 20 mg/dl, une valeur α-2 < 34 mg/dl, une valeur α-3 > 30, et une valeur α-4 > 25 mg/dl, où un profil de risque limite comprend une valeur α-1 comprise entre 20-30 mg/dl, une valeur α-2 comprise entre 34-45 mg/dl, une valeur α-3 comprise entre 13,5-30 mg/dl, et une valeur α-4 comprise entre 17-25 mg/dl, et où un profil de faible risque comprend une valeur α-1 > 30, une valeur α-2 > 45, une valeur α-3 < 13,5, et une valeur α-4 < 25 ;
la comparaison dudit profil de sous-populations de HDL avec le profil de sous-populations de HDL de référence pour ainsi déterminer un risque d'accumulation de plaque chez le sujet féminin.

6. Procédé selon la revendication 5, dans lequel les sous-populations de HDL comprennent en outre des particules pré-β-1 et le profil de risque accru comprend en outre une valeur pré-β-1 > 25 mg/dl.

7. Procédé selon la revendication 5, dans lequel les sous-populations de HDL comprennent en outre des particules pré-β-1 et le profil de risque limite comprend en outre une valeur pré-β-1 comprise entre 17-25 mg/dl.

8. Procédé selon la revendication 5, dans lequel les sous-populations de HDL comprennent en outre des particules pré-β-1 et la valeur du profil de faible risque comprend une valeur pré-β-1 < 17 mg/dl.
